(19) Europäisches Patentamt
European Patent Office
Office européen des brevets

(11) Veröffentlichungsnummer: **0 110 432**
**B1**

(12) # EUROPÄISCHE PATENTSCHRIFT

(45) Veröffentlichungstag der Patentschrift: **04.12.85**

(51) Int. Cl.⁴: **C 07 C 61/39,** C 07 C 51/00

(21) Anmeldenummer: **83201265.2**

(22) Anmeldetag: **02.09.83**

---

(54) **Verfahren zur Herstellung von Fluoren-9-carbonsäure.**

---

(30) Priorität: **27.11.82 DE 3243981**

(43) Veröffentlichungstag der Anmeldung: **13.06.84 Patentblatt 84/24**

(45) Bekanntmachung des Hinweises auf die Patenterteilung: **04.12.85 Patentblatt 85/49**

(84) Benannte Vertragsstaaten: **CH DE FR GB IT LI**

(56) Entgegenhaltungen: **CH - A - 286 853**

(73) Patentinhaber: **Rütgerswerke Aktiengesellschaft, Mainzer Landstrasse 217, D-6000 Frankfurt a.Main 1 (DE)**

(72) Erfinder: **Orth, Winfried, Dr., Am Schachtelgraben 28, D-6735 Hassloch/Pfalz (DE)**
Erfinder: **Pastorek, Emmerich, Dr., Grünberger Strasse 90, D-6944 Hemsbach (DE)**
Erfinder: **Fickert, Werner, Dr., Stockacher Strasse 14, D-6800 Mannheim (DE)**

**EP 0 110 432 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Fluoren-9-carbonsäure aus Fluoren.

Fluoren-9-carbonsäure ist ein wichtiges Zwischenprodukt bei der Herstellung bestimmter Antidiabetika oder Antiarrhythmika.

Zur Darstellung von Fluoren-9-carbonsäure gibt es eine Vielzahl von Verfahren, die teils von schwierig darzustellenden Fluorenderivaten und teils von Fluoren selbst ausgehen.

Die vom Fluoren ausgehenden Synthesen sind, wie z. B. in US-PS 3 692 826 beschrieben, im wesentlichen Umsetzungen mit Kohlendioxid in Anwesenheit von Kondensationsmitteln wie Natrium, Lithium, Butyllithium, Lithiumazid, Kaliumamid, Kaliumphenolat, $\alpha$-Phenyl-isopropyl-kalium, Bariumphenolat oder Diphenylharnstoff und Kaliumcarbonat. Diese Verfahrensweisen ergeben neben geringeren Ausbeuten auch niedrige Raumausbeuten. Die Verfahren sind somit nicht wirtschaftlich.

Daneben kennt man z. B. aus Chem. Ber. 44,206 (1911) oder aus J. Chem. Soc. 1949, 2623 die Umsetzung von Fluoren mit Oxalsäurederivaten. Bei diesen Synthesen werden die Synthesen in komplizierten, mehrfachen Reaktionsstufen durchgeführt, die für eine technische Produktion nicht geeignet sind.

Die Vielzahl der Syntheseversuche und auch die teils exotischen Darstellungsverfahren zeigen, daß ein Bedürfnis vorhanden war, neue Wege für die Herstellung dieser Verbindung zu erschließen.

Es bestand daher die Aufgabe, ein einfaches wirtschaftliches Verfahren zu finden, um aus dem leicht zugänglichen Fluoren in möglichst wenig, leicht zu handhabenden Verfahrensschritten in großer Ausbeute Fluoren-9-carbonsäure herzustellen.

Die Lösung erfolgt dadurch, daß Fluoren mit Dialkylcarbonat und Alkalihydrid oder Kaliumalkoholat umgesetzt, das Reaktionsgemisch mit einer Säure neutralisiert und der entstandene Fluoren-9-carbonsäureester isoliert und anschließend verseift wird.

Das als Ausgangspunkt eingesetzte Fluoren ist im Steinkohlen-Hochtemperaturteer im Durchschnitt zu etwa 2% enthalten. Es wird technisch durch Redestillation des Steinkohlenteer-Waschöls oder direkte Abnahme einer Fluoren-Fraktion bei der Primärdestillation des Steinkohlenteers und anschließende Umkristallisation, z. B. aus Lösungsbenzol, ggf. nach Entfernung von Phenolen und Basen, gewonnen.

Es wurde gefunden, daß dieses Fluoren einer gekoppelten Reaktion zugänglich ist, wenn es mit Dialkylcarbonat und Alkalihydrid oder Kaliumalkoholat bei mäßigen Temperaturen umgesetzt wird. Dabei entsteht das Fluoren der entsprechenden Fluoren-9-carbonsäureester gemäß der allgemeinen Gleichung

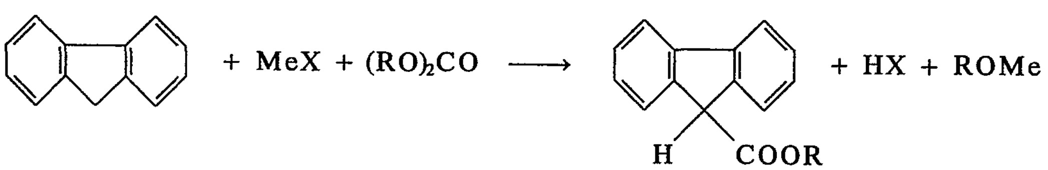


wobei R eine niedrige Alkylgruppe und X Wasserstoff oder einen Alkoholatrest darstellten. Me bedeutet Natrium oder Kalium, falls X Wasserstoff darstellt und Kalium, sofern X einen Alkoholatrest darstellt. Diese Umsetzung ist insofern überraschend, als mit dem normalerweise bei derartigen gekoppelten Reaktionen verwendeten Natriumamid keine Esterbildung stattfindet.

Als Fluoren kann ein technisches Produkt eingesetzt werden mit einer Reinheit von etwa 95%. Als Alkalihydrid kann gleichermaßen Kalium- oder Natriumhydrid verwendet werden, wobei z. B. als Natriumhydrid ein etwa 80%iges technisches Produkt verwandt wird. Die Menge des eingesetzten Alkalihydrids muß mindestens der stöchiometrisch notwendigen Menge entsprechen. Es wurde gefunden, daß die Reaktion mit besonders guter Ausbeute abläuft, wenn Alkalihydrid in einmolarem Überschuß zugegeben wird.

Erfindungsgemäß verwendbare Kaliumalkoholate sind die Kaliumsalze aller niederen Alkohole wie Methanol, Ethanol, Propanol, Isopropanol, Butanol und aller Isomere sowie Amylalkohol und aller Isomere. Wesentlich ist, daß die Alkoholate keinen freien Alkohol enthalten. Auch die Kaliumalkoholate werden mindestens in der notwendigen stöchiometrischen Menge eingesetzt, wobei auch hier durch einen Überschuß die Ausbeute verbessert wird.

Als Dialkylcarbonat kann ein reines Produkt eingesetzt werden, wie z. B. Dimethyl-, Diäthyl-, Dipropyl- oder Diisopropylcarbonat, wobei die entsprechenden Fluoren-9-carbonsäureester entstehen. Da diese Ester anschließend verseift werden, können auch Gemische verschiedener Dialkylcarbonate oder auch Mischcarbonate wie Äthyl-methyl-carbonat oder Äthyl-propyl-carbonat verwendet werden. Zweckmäßigerweise dient das eingesetzte Dialkylcarbonat sowohl als Reaktionspartner als auch als Lösungsmittel für das Fluoren. Dadurch ist immer Dialkylcarbonat im Überschuß vorhanden. Löst man das Fluoren in einem anderen Lösungsmittel wie Benzol, Toluol oder Xylol, so muß die zugegebene Menge an Dialkylcarbonat mindestens der stöchiometrisch notwendigen Menge entsprechen. Es wurde aber gefunden, daß eine wirtschaftliche Ausbeute erst bei einem mehr als vierfachen stöchiometrischen Überschuß des Dialkylcarbonats erzielt wurde. Bei mehr als fünfzehnfachem stöchiometrischem Überschuß wird die Reaktion infolge der großen Lösungsmittelmenge unwirtschaftlich.

Das Verfahren wird so durchgeführt, daß Natriumhydrid in einem aromatischen Lösungsmittel und/oder Dialkylcarbonat suspendiert und dazu eine Lösung von Fluoren in Dialkylcarbonat langsam zudosiert wird. Die Umsetzung erfolgt unter Rühren bei einer Temperatur im Bereich von 40—80°C während 2—8 Stunden. Danach wird abgekühlt und das Reaktionsgemisch unter Kühlen mit einer Säure, zweckmäßigerweise einer wäßrigen Lösung einer starken anorganischen Säure, neutralisiert.

Danach wird von eventuellen Verunreinigungen abfiltriert, die wäßrige Phase abgetrennt und von der verbleibenden organischen Phase destillativ das Lösungsmittel oder Lösungsmittelgemisch abgetrennt. Der im Destillationsrückstand verbleibende Fluoren-9-carbonsäureester wird daraufhin sauer verseift. Dabei fällt die Fluoren-9-carbonsäure in Form von hellbeigen Kristallen aus und wird abgetrennt, gewaschen und getrocknet.

Die Reaktionsfolge wird durch die folgenden Beispiele noch verdeutlicht.

## Beispiel 1

974 g (8,2 Mol) Diethylcarbonat und 120 g (4 Mol) Natriumhydrid, 80%ig, werden vorgelegt und ~10% einer Lösung, die aus 332 g (1,9 Mol) Fluoren, ~95%ig, und 974 g (8,2 Mol) Diethylcarbonat hergestellt wurde, zugegeben und das Gemisch unter Rühren auf 65°C aufgeheizt. Nach Einsetzen der Wasserstoff-Entwicklung wird Stickstoff an die Oberfläche des Reaktionsgemisches geführt und die restlichen Lösung bei 65°C in 1 bis 1 1/2 Stunden zugegeben, noch 4 Stunden bei 60°C nachgerührt und auf 20°C abgekühlt.

Das Reaktionsgemisch läßt man vorsichtig zu einer Lösung von 400 g (4 Mol) Salzsäure, konz., und 500 g Wasser bei Wasserkühlung zufließen, so daß die Temperatur nicht über 40°C ansteigt. Das Gemisch wird von Verunreinigungen abfiltriert, die Wasserschicht abgetrennt und aus der Diethylcarbonat-Schicht das Lösungsmittel unter vermindertem Druck abdestilliert. Als Destillat werden ~1700 ml Diethylcarbonat erhalten. Zum Destillationsrückstand werden 1049 g Essigsäure, konz., und 420 g Salzsäure, 10%ig, zugegeben und 4 Stunden zum Rückfluß erhitzt. Nach ~1/2 Stunde fallen bei Siedetemperatur Kristalle der Fluorencarbonsäure aus. Nach beendeter Reaktion kühlt man das Reaktionsgemisch auf ~20°C ab, nutscht das ausgefallene kristalline Produkt ab, wäscht mit 600 ml Toluol und zum Schluß mit etwa 500 ml Wasser chloridfrei. Das erhaltene Naßprodukt (360—400 g) wird bei ~80°C getrocknet.

Es werden 322 g Fluoren-9-carbonsäure als hellbeiges kristallines Produkt (Fp. 226—230°C) erhalten. Die Ausbeute beträgt 80,7% der Theorie, bezogen auf eingesetztes Fluoren.

## Beispiel 2

Es wird, wie in Beispiel 1 angeführt, verfahren. Jedoch werden anstelle von 974 g Diethylcarbonat 1 l Toluol vorgelegt.

Es werden 330 g Fluoren-9-carbonsäure als hellbeiges kristallines Produkt erhalten (Fp = 226—230°C).

Die Ausbeute beträgt 82,7% der Theorie, bezogen auf eingesetztes Fluoren.

## Beispiel 3

584 g (4,6 Mol) Diethylcarbonat und 186 g Kaliumethylat (2,2 Mol) werden vorgelegt und unter Kühlung wird eine Lösung, die aus 332 g (1,9 Mol) Fluoren, ~95%ig und 974 g (8,2 Mol) Diethylcarbonat hergestellt wurde, so zugegeben, daß die Temperatur von 40°C nicht überschritten wird. Danach wird noch 5 Stunden bei 65—70°C gerührt und auf 20°C abgekühlt.

Das Reaktionsgemisch läßt man vorsichtig zu einer Lösung von 220 g (2,2 Mol) Salzsäure und 500 g Wasser unter Kühlung zufließen, so daß die Temperatur nicht über 40°C ansteigt. Die weitere Aufarbeitung erfolgt analog Beispiel 1.

Es werden 280 g Fluoren-9-carbonsäure als hellbeiges kristallines Produkt (Fp = 227—29°C) erhalten. Die Ausbeute beträgt 72,7% der Theorie.

**Patentansprüche**

1. Verfahren zur Herstellung von Fluoren-9-carbonsäure aus Fluoren, dadurch gekennzeichnet, daß Fluoren mit mindestens stöchiometrischen Mengen Dialkylcarbonat mit Alkylketten von wahlweise 1 bis 3 C-Atomen und Alkalihydrid oder Kaliumalkoholat umgesetzt, das Reaktionsgemisch mit einer Säure neutralisiert und der entstandene Fluoren-9-carbonsäureester isoliert und anschließend verseift wird.

2. Verfahren nach Anspruch 1, dadurch gekennzeichnet, daß Natriumhydrid in einmolarem Überschuß eingesetzt wird.

3. Verfahren nach Anspruch 1 und 2, dadurch gekennzeichnet, daß die Umsetzung von Fluoren mit Dialkylcarbonat und Alkylihydrid oder Kaliumalkoholat bei einer Temperatur im Bereich von 40 bis 80° C durchgeführt wird.

4. Verfahren nach den Ansprüchen 1—3, dadurch gekennzeichnet, daß das Dialkylcarbonat in einem mehr als vierfachen Überschuß zugegeben wird.

## Claims

1. A process for the preparation of fluorene-9-carboxylic acid, characterized in, that fluorene is reacted with at least stoichiometric amounts of dialkyl carbonate having alkyl chains of 1 to 3 carbon atoms and alkali metal hydride or potassium alcoholate, that the reaction mixture is neutralized with an acid and the resulting fluorene-9-carboxylic acid ester is isolated and saponified.

2. The process of claim 1 wherein sodium hydride is present in a one molar excess.

3. The process of claims 1 and 2 wherein the reaction of fluorene with dialkyl carbonate and alkali metal hydride or potassium alcoholate is effected at a temperature in the range of 40 to 80° C.

4. The process of claims 1 to 3 wherein the dialkyl carbonate is used in at least a 4 molar excess.

## Revendications

1. Procédé de préparation d'acide 9-fluorène carboxylique à partir de fluorène, caractérisé par le fait qu'on fait réagir du fluorène avec des quantités au moins stoechiométriques de carbonate de dialcoyle ayant des chaînes alcoyles de 1 à 3 atomes de C au choix et avec de l'alcoolate de potassium, qu'on neutralise le mélange réactionnel avec un acide et qu'on isole le 9-fluorène carboxylate formé et qu'on le saponifie ensuite.

2. Procédé selon la revendication 1, caractérisé en ce que l'hydrure de sodium est mis en œuvre selon un excès d'une mole.

3. Procédé selon les revendications 1 et 2, caractérisé en ce qu'on réalise la réaction du fluorène avec du carbonate de dialcoyle et de l'hydrure alcalin ou de l'alcoolate de potassium à une température située dans un domaine de 40 à 80° C.

4. Procédé selon les revendications 1 à 3, caractérisé en ce qu'on ajoute le carbonate de dialcoyle en un excès de plus de quatre fois.